# EUROPÄISCHE PATENTSCHRIFT

(11) **EP 1 792 999 B1**
(45) Veröffentlichungstag und Bekanntmachung des Hinweises auf die Patenterteilung: **18.03.2009**
(21) Anmeldenummer: 06076503.9
(22) Anmeldetag: 28.07.2006
(51) Int. Cl.: C12P 7/64

(54) **Verfahren zur enzymatischen Synthese von Triglyceriden**
Process for the enzymatic synthesis of triglycerides
Procédé pour la synthèse enzymatique de triglycérides

(30) Priorität: 03.12.2005 DE 102005057832
(43) Veröffentlichungstag der Anmeldung: 06.06.2007
(73) Patentinhaber: Cognis IP Management GmbH, 40589 Düsseldorf (DE)
(72) Erfinder: Schörken, Ulrich, 40591 Düsseldorf (DE); Meyer, Carolin, 40589 Düsseldorf (DE); Horlacher, Peter, 89287 Bellenberg (DE); Both, Sabine, 41470 Neuss (DE)

(56) Entgegenhaltungen:
- EP-A- 1 582 594
- EP-A- 1 582 595
- EP-A1- 1 411 129
- WO-A-02/24935
- WO-A-91/16443
- WO-A-94/26855
- US-A- 5 219 744
- ROBLES MEDINA A ET AL: "Lipase-catalyzed esterification of glycerol and polyunsaturated fatty acids from fish and microalgae oils" JOURNAL OF BIOTECHNOLOGY, ELSEVIER SCIENCE PUBLISHERS, AMSTERDAM, NL, Bd. 70, Nr. 1-3, 30. April 1999 (1999-04-30), Seiten 379-391, XP004173418 ISSN: 0168-1656

## Beschreibung

### Gebiet der Erfindung

Die Erfindung befindet sich auf dem Gebiet der Fettsäureester und betrifft ein neues Verfahren zur enzymatischen Synthese von Estern mehrfach ungesättigter, kurzkettiger oder empfindlicher Fettsäuren mit einem hohen Triglyceridanteil, das sich aus einer enzymatischen Synthese des Gemisches aus Triglyceriden und Partialglyceriden ausgehend von freier Säure bzw. deren Alkylestern und Glycerin und einer anschliessenden selektiven enzymatischen Rückhydrolyse der Partialglyceride zu Fettsäure und Glycerin, sowie einer destillativen Trennung der Triglyceride von Fettsäuren und / oder überschüssigen Alkylestern und Glycerin zusammensetzt, sowie die durch dieses Verfahren erhältlichen triglyceridreichen Estergemische.

### Stand der Technik

Die chemische Synthese von Glycerid-Estern mehrfach ungesättigter Fettsäuren und empfindlicher Fettsäuren hat den Nachteil, dass in der Regel sehr hohe Temperaturen eingesetzt werden müssen und große Mengen an chemischen Katalysatoren benötigt werden, wodurch in höherem Maße Nebenprodukte und unerwünschte Isomerisierungen auftreten. Enzymatisch katalysierte Umsetzungen mit Lipasen finden in der Regel unter milderen Bedingungen statt und ergeben Endprodukte hoher Reinheit. Auch gesättigte Fettsäuren und deren Alkylester wie zum Beispiel mittelkettige Fettsäuren (insbesondere C8 und C10), die aus Palmkern- und Kokosöl gewonnen werden, setzen bei Hochtemperatursynthesen Farbe und Geruch frei. Insbesondere aus Kokosöl gewonnene Fettsäuren und Ester neigen zu Farbbildung. Auch hier kann eine enzymatische Synthese unter milden Bedingungen zu farblich und geruchlich besseren Produkten führen.

In der Europäischen Patentanmeldung EP1 322 776A1 wird eine lipase katalysierte Methode zur Herstellung von Triglyceriden mehrfach ungesättigter konjugierter Fettsäuren ausgehend vom Alkylester der ungesättigten Fettsäuren und Glycerin beschrieben, die den entstehenden Alkohol unter vermindertem Druck aus der Reaktion entfernt. Auch aus der Internationalen Patentanmeldung WO9116443A1 ist die Veresterung von Glycerol und freien mehrfach ungesättigten Fettsäuren oder deren Alkylestern zu den entsprechenden Triglyceriden durch Entfernung des Reaktionswassers bzw. des entstehenden Alkohols unter vermindertem Druck beschrieben.

EP-A 1 582 594 offenbart Fettsäureester mehrfach ungesättigter Fettsäuren mit einem Triglyceridanteil von mindestens 90% und Verfahren zu deren Herstellung.

EP-A 1 411 129 Fettsäureester mittelkettiger Fettsäuren (z.B. Caprylsäure) mit einem Triglyceridanteil von mindestens 90% und Verfahren zu deren Herstellung.

US-A 5,219,744 offenbart die selektive Hydrolyse von Partialglyceriden, insbesondere Diglyceriden.

WO 94/26855 offenbart ein Verfahren zur enzymkatalysierten Umesterung von Triglyceridgemischen.

Enzymatische Synthesen haben jedoch häufig den Nachteil, dass die Umsetzungen relativ langsam ablaufen.

Insbesondere eine Erhöhung des Triglyceridgehaltes von z.B. 90 % auf 95 % oder noch höher ist sehr zeit- und damit kostenintensiv, da die Reaktionsgeschwindigkeit der enzymatischen Synthese der Michaelis-Menten Kinetik gehorcht und proportional zur Konzentration der Edukte und Produkte ist. Eine Möglichkeit, den Gehalt an Triglyceriden ohne eine deutlich längere Reaktionszeit in Anspruch zu nehmen ist die destillative Abtrennung der Fettsäuren und Partialglyceride von den Triglyceriden. Es zeigt sich allerdings, dass die Abtrenung von Diglyceriden kaum möglich ist, da sehr hohe Destillationstemperaturen benötigt werden, die zu einer Beeinträchtigung der Produktqualität des Triglyceride führen.

Die Aufgabe der vorliegenden Erfindung hat daher darin bestanden, eine verbesserte Methode zur Herstellung von Estern mehrfach ungesättigter, kurzkettiger oder empfindlicher, insbesondere mehrfach ungesättigter Fettsäuren mit einem hohen Triglyceridanteil mit hoher Ausbeute und in kurzer Reaktionszeit zu erhalten.

### Beschreibung der Erfindung

Gegenstand der Erfindung ist ein Bitte Verfahren zur enzymkatalysierten Synthese von Estern von Fettsäuren mit hohem Triglyceridanteil bei dem man
(a) eine Synthese der Fettsäuren oder ihrer Alkylester mit Glycerin in ihre Triglyceride in Gegenwart eines Enzyms unter Vakuum und wasserarmen Bedingungen durchführt
(b) im Anschluss die im Schritt a) ebenfalls entstandenen Partialglyceride in Gegenwart eines Enzyms nach Zugabe von Wasser unter Normaldruck rückhydrolysiert und
(c) Fettsäuren bzw. Alkylester und Glycerin destillativ oder über Raffination vom Triglycerid abtrennt,
wobei man kurzkettige Fettsäuren mit 6 bis 12 Kohlenstoffatomen und/oder mehrfach ungesättigte Fettsäuren auswählt aus der Gruppe, die gebildet wird von Docosahexaensäure, Eicosapentaensäure, Linolsäure und konjugierter Linolsäure in Form der freien Säure oder in Form der Methyl- oder Ethylester einsetzt, und wobei man immobilisierte Candida antarctica B Lipase sowohl für Synthese als auch Rückhydrolyse einsetzt.

Die Synthese und die nachfolgende selektive Rückhydrolyse der Partialglyceride werden in der Regel in einem Eintopfverfahren im selben Reaktor durchgeführt.

Die enzymatische Synthese von Fettsäure-Glyceridestern (Schritt a) mit hohem Triglyceridanteil erfordert in der Regel lange Reaktionszeiten, die eine Belastung für empfindliche Fettsäuren darstellt. Es wurde nun überraschender Weise festgestellt, dass man den Triglyceridanteil dadurch erhöhen kann, dass man eine selektive Rückhydrolyse (Schritt b) der im Schritt a) entstandenen Partialglyceride direkt im Anschluss an a) durchrühren kann, da die Partialglyceride leichter als die Triglyceride rückhydrolysieren.

Die so hergestellten Estermischungen zeichnen sich durch eine gute Reinheit, wenig Nebenprodukte und eine gute Stabilität aus, da die empfindlichen Fettsäuren durch die geringe Belastung bei verringerter Reaktionszeit keinen Schaden genommen haben. Auf diese Weise ist es möglich in einer kurzen Reaktionszeit von 2 bis 25 Stunden chemisch hochwertige Estermischungen mit einem Triglyceridanteil von mindestens 90 %, vorzugsweise mindestens 95 % und sogar mindestens 98 % bezogen auf den Anteil der Gesamtglyceride zu synthetisieren.

Das Verfahren ist besonders gut anwendbar zur Synthese von Estergemischen ungesättigter Fettsäuren mit hohem Triglyceridanteil, zur Synthese von MCT-Ölen oder zur Synthese strukturierter Lipide.

### Vorteile von triglyceridreichen Estern gegenüber 1,3-Diglyceriden bei wirksamen Fettsäuren (CLA, PUFAs):

Die meisten Lipasen, die zur Synthese von Triglyceriden gut geeignet sind wie z.B. Candida antarctica B Lipase oder Rhizomucor miehei Lipase bevorzugen stark die 1- und 3-Position des Glycerins. Daher verläuft die Synthese hauptsächlich über 1-Monoglycerid und 1,3-Diglycerid als Intermediates. 2-Monoglycerid und 1,2-Diglycerid entstehen dabei nur über Acylmigration und Nebenreaktion und werden schnell weiter zum Triglycerid synthetisiert. Nach Beendigung der Synthesereaktion sind daher 1-Mono sowie 1,3-Diglycerid die Haupt-Nebenkomponenten im Produkt.

Die Fettsäuren, die an den Positionen 1 und 3 des Glycerids gebunden sind, werden anders verstoffwechselt als die an 2-Position gebundenen Fettsäuren. Die 1- und 3- gebundenen Fettsäuren werden im Darm abgespalten, wobei das 2-Monoglycerid erhalten bleibt. Die Fettsäuren und das 2-Monoglycerid werden adsorbiert und Triglycerid wird aus 2-Monoglycerid und Fettsäuren resynthetisiert. Ein Teil der Fettsäuren wird anderweitig verstoffwechselt, zum Beispiel findet ein Transport über die Blutbahn und oxidativer Abbau zur Energieerzeugung statt.

Auf dem Prinzip des unterschiedlichen Abbaus und Transports von 2-gebundenen und 1,3-gebundenen Fettsäuren beruht beispielsweise die dietätische Wirkung von Enovaöl, einem synthetischem Diglycerid mit hohem 1,3-Diglycerid Anteil, bei dem nur wenig 2-Monoglycerid zur Resynthese von Triglycerid zur Verfügung steht. Da nur wenig Resynthese stattfindet, werden weniger Fette in den Fettzellen eingelagert sondern die aufgenommenen Fettsäuren zu einem größeren Teil in Energie umgewandelt.

Bei der Aufnahme von Triglyceriden, die pharmakologisch wirksame Fettsäuren wie CLA (konjugierte Linolsäure), EPA (Eicosapentaensäure) oder DHA (Docosahexaensäure) enthalten, führt eine oxidative Verbrennung zu einer Erniedrigung der Dosis bezogenen Wirkung. Daher sind 1,3-Diglyceride aus oben beschriebenen Gründen eine weniger geeignete Form zur Verabreichung dieser Fettsäuren, sondern Triglyceride sind zu bevorzugen. Aus diesem Grund ist eine Verringerung des Gehaltes an 1,3-Diglycerid durch enzymatische selektive Rückhydrolyse in Triglyceriden, die pharmakologisch wirksame Fettsäuren enthalten, zu empfehlen, insbesondere wenn keine weiteren fetthaltigen Speisen aufgenommen werden.

Im Fall von CLA als wirksamer Fettsäure ist ein Wirksamkeitsunterschied zwischen freier Fettsäure und Triglycerid in einer Langzeitstudie nachgewiesen worden (Gaullier et al., 2004; AJCN 79; 1118-1125). Die Gabe von Triglycerid führte dabei nach 12 Monaten zu einer verbesserten Reduktion der BFM (Body Fat Mass) im Vergleich zur freien Säure. Die Wirkung von freier Säure sollte dabei vergleichbar sein mit der Wirkung eines 1,3-Diglycerids, da dieses im Darm komplett in Glycerin und die freien Säuren gespalten wird. Indirekt ist also aus dieser Studie abzuleiten das ein CLA-Glycerid eine besonders gute Wirkung zeigt, wenn der Gehalt an Triglycerid sehr hoch ist und der Gehalt an 1,3-Diglycerid niedrig ist.

### Anwendung zur Erreichung hoher OH-Zahl Spezifikationen bei Myritolen, deren Direktsynthese schwierig ist:

MCTs (Middle Chain Triglycerides) sind Triglyceride, die als Hauptkomponenten Caprylsäure und Caprinsäure enthalten. In geringen Mengen sind Capronsäure oder Laurinsäure gebunden. Diese werden eingesetzt in der Kosmetik als Emollient oder als Medical Food bei Patienten mit Erkrankungen des Fettstoffwechsels. Für beide Anwendungen exisitieren hohe Anforderungen an Geruch, Farbe und Gehalt an freien Hydroxylgruppen. Um den Anforderungen zu entsprechen müssen nach einer chemischen Synthese Aufarbeitungsschritte erfolgen um insbesondere die Geruchs- und Farbanforderungen zu erfüllen.

Mit einer enzymatischen Synthese lassen sich bei niedrigen Temperaturen qualitativ bessere Produkte herstellen. Allerdings ist es schwierig die Anforderungen an den Gehalt freier Hydroxylgruppen in einer direkten Synthese zu erfüllen. So liegt zum Beispiel die OH-Zahl Spezifikation für Myritol (MCT von Cognis) bei maximal 5. Hier kann eine OH-Zahl Verbesserung durch selektive Rückhydrolyse von Mono- und Diglyceriden, die frei OH-Gruppen besitzen, erreicht werden. Freies Glycerin und Fettsäure können leicht vom MCT abgetrennt werden.

### Anwendung bei strukturierten Lipiden:

In jüngster Zeit werden vermehrt strukturierte Lipide, die zumeist aus Mischungen von mittelkettigen und essentiellen Fettsäuren bestehen, in der Literatur untersucht. Das hier beschriebene Verfahren ist auch geeignet zur Erhöhung des Triglyceridgehaltes in diesen strukturierten Lipiden, da jede der Einzelkomponenten von den Enzymen erkannt werden uns somit auch aus diesen Lipiden selektiv die Mono- und Diglyceride hydrolysiert werden.

Insbesondere aus strukturierten Lipiden, die mittelkettige Fettsäuren enthalten, sollten die Partialglyceride möglichst vollständig entfernt werden, da die Rauchpunkte isb. von mittelkettigen Partialglyceriden sehr niedrig sind. Strukturierte Lipide mit hohen Anteilen an Partialglyceriden sind damit nicht zum Braten und Frittieren geeignet.

### 1. Schritt (a) - Enzymatische Synthese des Gemisches aus Triglyceriden und Partialglyceriden

Der erste Schritt des Eintopfverfahrens, die Synthese der Triglyceride, wird unter wasserfreien Bedingungen gefahren. Bei der Auswahl von freien Fettsäuren als Ausgangmaterial hat sich der Zusatz von Salzen, vorzugsweise Natriumcarbonat zur Beschleunigung der Synthese bewährt. Diese können trocken oder gelöst in Wasser dem Ansatz zugegeben werden, so dass im letzteren Fall als Ausnahme sehr geringe Mengen Wasser dem Ansatz zugefügt werden, die unter 1 % vorzugsweise unter 0,5 % bezogen auf den Gesamtansatz liegen.

Die Synthese wird unter Vakuum zur Entfernung von Reaktionswasser durchgeführt, dass beim Einsatz freier Säuren gebildet wird oder zur Entfernung von Alkohol, dass beim Einsatz der entsprechenden Ester, bevorzugt Methyl- oder Ethylester, gebildet wird. Dadurch wird das Reaktionsgleichgewicht zur Glyceridsynthese hin verschoben.

Es wird Candida antarctica B Lipase immobilisiert auf Trägermaterial eingesetzt, insbesondere 3 bis 12 Gew. % Immobilisat bezogen auf den Fettanteil. Für das erfindungsgemäße Verfahren eignen sich verschiedenste Trägermaterialien, die zur Bindung von Enzymen geeignet sind. Als Träger können Kunststoffe, mineralische Träger oder Harze eingesetzt werden, die die Esterasen über hydrophobe Wechselwirkungen binden wie z.B. Amberlite 16 (Rohm & Haas), Celite oder Accurel MP 1000 (Membrana). Als weiteres eignen sich Ionentauscher, die die Esterasen über ionische und zum Teil auch hydrophobe Wechselwirkungen binden wie z.B. Dowex Marathon WBA (Dow Chemicals) oder Duolite A 568 (Rohm & Haas). Als weiteres geeignet sind Träger, die Esterasen über chemisch reaktive Gruppen binden können wie z.B. Eupergit (Degussa).

Als weiteres eignen sich chemische Modifizierungen für die Adaption der Esterasen auf das Reaktionssystem. Hierbei können hydrophobe Modifizierungen wie zum Beispiel Coating mit Surfactants oder chemische Modifizierung mit Fettaldehyden zum Einsatz kommen. Ebenfalls geeignet ist die Stabilisierung der Esterasen über Quervernetzung zum Beispiel durch Glutaraldehyd, DMA oder EDC.

Als weiteres eignet sich die Kombination aus chemischer Modifizierung und Immobilisation zur Adaption der Lipase auf das Reaktionssystem. Dabei kann entweder die Lipase zuerst immobilisiert werden und anschliessend trägergebunden modifiziert werden oder bereits chemisch modifizierte Lipase wird immobilisiert.

Der für die Reaktion geeignete Temperaturbereich richtet sich nach dem Aktivitätsoptimum des Enzyms. Für die ausgewählte Lipase hat sich 40 bis 90° C als besonders geeignet erwiesen, speziell bevorzugt wird der Bereich von 60 bis 80°C. Dabei sollte ein Vakuum von mindestens 200 mbar, bevorzugt 1 bis 100 mbar und besonders bevorzugt 5 bis 60 mbar angelegt werden. Die bevorzugten Versuchsparameter ergeben sich aus der zu erreichenden Beschleunigung der Reaktionsgeschwindigkeit.

### 2. Schritt (b) - Selektive Hydrolyse der Partialglyceride:

Nach der wasserarmen enzymatischen Synthese werden im gleichen Reaktionsgefäss in wasserreichem Medium bei niedrigeren Temperaturen die entstandenen Partialglyceride in Alkohol und die freie Säure gespalten. Unter "wasserreich" wird eine Wassermenge von maximal 50 %, vorzugsweise maximal 25 % besonders bevorzugt maximal 20 % und mehr als 1 % vorzugsweise mindestens 2 %, besonders bevorzugt mindestens 5 % Wasser bezogen auf den Gesamtansatz verstanden.

Der geeignete Temperaturbereich der selektiven Rückhydrolyse beträgt in der Regel 20 - 60°C, bevorzugt 20 - 45°C. Er ist somit niedriger als der Temperaturbereich der Synthese, der normalerweise 10 bis 80°C, vorzugsweise 30 bis 50°C höher als der des zweiten Verfahrensschrittes ist.

Die Hydrolyse findet bei Normaldruck unter Rühren statt. Bei Einsatz von immobilisiertem Enzym wird dieses nach der Hydrolyse über Filtration abgetrennt.

### 3. Schritt (c) - Destillative Trennung der Triglyceride von Fettsäuren und Glycerin

Die Aufarbeitung erfolgt durch die Abtrennung der wässrigen Phase durch Zentrifuge oder Abscheider, ggf. unter vorherigem leichtem Ansäuern zur besseren Phasenseparation und zum Neutralisieren von alkalischen Zusätzen, die in der Synthese eingesetzt wurden. In einem alternativen Verfahren wird das zugesetzte Wasser destillativ aus dem Produktgemisch abgetrennt.

Danach wird eine Destillation von Fettsäure und / oder überschüssigem Fettsäurealkylester sowie ggf. noch enthaltenem Monoglycerid unter Vakuum durchgeführt, so dass das Sumpfprodukt die angereicherten Triglyceride enthält. Die abgetrennten Fettsäuren und / oder Fettsäurealkylester sowie ggf. vorhandenes freies Glycerin oder Monoglyceride können dann erneut zur Triglyceridsynthese eingesetzt werden.

### Beispiele

### Beispiel 1

### Enzymscreening zur selektiven Hydrolyse von Diglyceriden in kommerziell erhältlicher CLA-TG Präparation (Tonalin TG 80)

13 Reaktionsgefässe wurden mit jeweils 20 g Tonalin TG 80 und 2 g Wasser befüllt. Zu den Ansätzen wurde jeweils eine kommerziell erhältliche Enzympräparation wie in der Tabelle 1 unten aufgeführt zugegeben. Die Ansätze wurden bei Raumtemperatur unter Rühren inkubiert. Nach 1,5 h und 5 h wurden jeweils Proben entnommen, die Ölphase wurde über Zentrifugation abgetrennt und die Verteilung der Glyceride wurde gaschromatographisch analysiert. Angegeben ist das Verhältnis aus Triglyceriden bezogen auf die Menge an Gesamtglyceriden. Das Ausgangsprodukt Tonalin TG 80 hat einen Triglyceridgehalt von 81 % bezogen auf die Menge an Gesamtglyceriden. Die Auswertung erfolgt über Flächenprozent. Zusätzlich wurde von jeder Probe eine Säurezahl gemessen.

**Tabelle 1: Enzymscreening zur selektiven Hydrolyse von Diglyceriden in CLA-TG**

| **Ansatz** | **Enzym** | **Hersteller** | **Organismus** | **Menge** |
|---|---|---|---|---|
| 1 | Lipase A | Amano | Aspergillus nig. | 100 mg |
| 2 | Novozym 735 | Novozymes | Candida ant. A | 100 µl |
| 3 | Novozym 525 | Novozymes | Candida ant. B | 100 µl |
| 4 | Lipomod 34 | Biocatalysts | Candida cyl. | 20 mg |
| 5 | Lipase M | Amano | Mucor | 50 mg |
| 6 | Novozym 388 | Novozymes | Rhizomucor | 50 µl |
| 7 | Lipase G | Amano | Penicilium cam. | 20 mg |
| 8 | Lipase R | Amano | Penicilium roq. | 50 mg |
| 9 | Lipase L115 | Biocatalysts | Porcine pancreas | 50 mg |
| 10 | Lipase PS | Amano | Pseudomonas | 20 mg |
| 11 | Lipomod 36 | Biocatalysts | Rhizopus | 50 mg |
| 12 | Lipase FAP-15 | Amano | Rhizopus | 20 mg |
| 13 | Lipozym TL 100 | Novozymes | Thermomyces | 20 µl |

| **Ansatz** | **SZ (1,5 h)** | **% TG/Gesamt-glyceride (5 h)** | **SZ (5 h)** | **% TG/Gesamt glyceride (5h)** |
|---|---|---|---|---|
| 1 | 32 | 74 | 57 | 60 |
| 2 | 43 | 60 | 46 | 60 |
| 3 | 14 | 94 | 13 | 98 |
| 4 | 119 | 22 | 129 | 22 |
| 5 | 18 | 79 | 16 | 82 |
| 6 | 18 | 87 | 18 | 87 |
| 7 | 41 | 91 | 44 | 94 |
| 8 | 7 | 86 | 10 | 82 |
| 9 | 2 | 85 | 3 | 87 |
| 10 | 80 | 34 | 86 | 36 |
| 11 | 66 | 37 | 65 | 41 |
| 12 | 71 | 45 | 71 | 29 |
| 13 | 9 | 87 | 16 | 88 |

### Ergebnis:

Im Enzymscreening ist zu erkennen, dass Candida antarctica B Lipase (Novozym 525) und Penicilium camenbertii Lipase (Lipase G) den Gehalt an Triglycerid im kommerziell erhältlichen Tonalin TG 80 von 81 % signifikant auf bis zu 98 % bzw. 94 % erhöhen. Eine leichte Erhöhung des Triglyceridgehaltes wurde bei den Lipasen Novozym 388, Lipase R, Lipase L115 und Lipozym TL 100 detektiert.

### Beispiel 2

### Anreicherung von Triglycerid in Abhängigkeit der Enzymkonzentration von Novozym 525 und Lipase G und in Abhängigkeit der eingesetzten Wassermenge.

6 Reaktionsgefässe wurden mit jeweils 20 g Tonalin TG befüllt. Zu den Ansätzen wurde jeweils Wasser sowie Enzym wie in der Tabelle 2 aufgeführt zugegeben. Die Ansätze wurden bei Raumtemperatur unter Rühren inkubiert. Nach 1 h, 5 h und 22 h wurden jeweils Proben entnommen, die Ölphase wurde über Zentrifugation abgetrennt und die Verteilung der Glyceride wurde gaschromatographisch analysiert. Angegeben ist das Verhältnis aus Triglyceriden bezogen auf die Menge an Gesamtglyceriden. Das Ausgangsprodukt Tonalin TG 80 hat einen Triglyceridgehalt von 81 % bezogen auf die Menge an Gesamtglyceriden. Die Auswertung erfolgt über Flächenprozent. Zusätzlich wurde von jeder Probe eine Säurezahl gemessen.

**Tabelle 2: Anreicherung von Triglycerid in Abhängigkeit der Enzymkonzentration von Novozym 525 und Lipase G und in Abhängigkeit der eingesetzten Wassermenge**

| **Ansatz** | **Wasser** | **Enzym** | **SZ (1 h)** | **%TG/Gesamt-glyceride 1 h** |
|---|---|---|---|---|
| 1 | 0,5 g | 50 µl Novozym 525 | 17 | 96 |
| 2 | 0,5 g | 100 µl Novozym 525 | 18 | 98 |
| 3 | 1 g | 100 µl Novozym 525 | 25 | 96 |
| 4 | 0,5 g | 2 mg Lipase G | 15 | 88 |
| 5 | 0,5 g | 5 mg Lipase G | 15 | 90 |
| 6 | 1 g | 5 mg Lipase G | 30 | 93 |

| **Ansatz** | **SZ (5 h)** | **% TG/Gesamt-glyceride 1 h** | **SZ (22 h)** | **%TG/Gesamt-glyceride 22 h** |
|---|---|---|---|---|
| 1 | 18 | 97 | 17 | 98 |
| 2 | 16 | 99 | 17 | 99 |
| 3 | 23 | 96 | 23 | 95 |
| 4 | 24 | 88 | 19 | 89 |
| 5 | 23 | 92 | 21 | 96 |
| 6 | 36 | 94 | n.d. | 97 |

### Ergebnis:

In allen Ansätzen wird eine deutliche Anreicherung des Triglyceridgehaltes erreicht. Der hjöchste Triglyceridanteil wurde mit Novozym 525 bei einem Wassergehalt von 2,5 % und einer Enzymkonzentration von 0,5 % erreicht.

### Beispiel 3

### Synthese von CLA Triglycerid gekoppelt mit selektiver Hydrolyse der Partialglyceride

In einen Kolben werden 85 g CLA freie Säure, 9 g Glycerin, 6 g immobilisierte Candida antarctica B Lipase und 0,2 g Natriumcarbonat vorgelöst in 0,4 g Wasser gegeben. Bei einer Temperatur von 70 °C und einem Vakuum von 60 mbar wird der Ansatz unter Rühren inkubiert. Nach 24 h wird das Vakuum abgestellt und eine Probe wird zur gaschromatographischen Analyse entnommen. Die Temperatur wird auf 45 °C abgesenkt und 20 g Wasser werden zugegeben. Der Ansatz wird für 2 h inkubiert, wobei nach 1 h und 2 h Proben zur gaschromatographischen Analyse genommen werden. Anschliessend wird die Wasserphase durch Zentrifugation bei 12.000 upm von der Ölphase getrennt. Die Glyceridverteilung der Ölphase wird nach der Separation gaschromatographisch analysiert.

**Tabelle 3: Synthese von CLA -TG gekoppelt mit selektiver Hydrolyse der Partialglyceride**

| **Probe** | **Fettsäure [%]** | **Monoglyc. [%]** | **Diglycerid [%]** | **Triglycerid [%]** | **% Triglyc/ Gesamtglyc.** |
|---|---|---|---|---|---|
| Ausgangsgemisch | 100 | 0 | 0 | 0 | 0 |
| 24 h Synthese | 9,2 | 0 | 20,8 | 70,0 | 77 |
| 1 h Hydrolyse | 27,8 | 0 | 4,5 | 67,7 | 94 |
| 2 h Hydrolyse | 28,3 | 0 | 3,2 | 68,5 | 96 |
| Nach Separation | 28,6 | 0 | 2,9 | 68,5 | 96 |

### Ergebnis:

Die enzymatische Synthese von CLA-Triglycerid kann direkt mit der selektiven Rückhydrolyse der Partialglyceride in einer Eintopfreaktion kombiniert werden, sofern Candida antarctica B Lipase eingesetzt wird. Die gekoppelte Synthese / Hydrolyse Reaktion führt in einer deutlich schnelleren Zeit zu Triglyceridgehalten von > 95 % als die direkte enzymatische Synthese.

### Beispiel 4

### Synthese von Middle Chain Triglycerid (MCT) gekoppelt mit selektiver Hydrolyse der Partialglyceride

In einen Kolben werden 100 g Caprylsäure, 19 g Glycerin, 5 g immobilisierte Candida antarctica B Lipase und 0,2 g Natriumcarbonat vorgelöst in 0,4 g Wasser gegeben. Bei einer Temperatur von 70 °C und einem Vakuum von 60 mbar wird der Ansatz unter Rühren und Stickstoffbegasung inkubiert. Nach 45 h wird das Vakuum abgestellt und eine Probe wird zur gaschromatographischen Analyse entnommen. Das immobilisierte Enzym wird abfiltriert. In 2 Ansätze werden jeweils 10 g des MCT-Gemisches und 0,5 g Wasser eingewogen. Dazu werden in Ansatz 1 10 µl Novozym 525 und in Ansatz 2 1 mg Lipase G gegeben. Die beiden Ansätze werden für 24 h bei Raumtemperatur unter Rühren inkubiert. Nach 1 h, 5 h und 24 h werden Proben zur gaschromatographischen Analyse der Glyceridverteilung genommen.

**Tabelle 4: Synthese von Middle Chain Triglycerid (MCT) gekoppelt mit selektiver Hydrolyse der Partialglyceride**

| **Probe** | **Fettsäure [%]** | **Monoglycerid [%]** | **Diglycerid [%]** | **Triglycerid [%]** | **% Trigly/ Gesamtglyc.** |
|---|---|---|---|---|---|
| Ausgangsgemisch | 100 | 0 | 0 | 0 | 0 |
| 45 h Synthese | 15,2 | 0 | 4,2 | 80,5 | 95 |

| Ansatz 1 | | | | | |
|---|---|---|---|---|---|
| 1 h Hydrolyse | 17,3 | 0,4 | 1,5 | 80,8 | 97,7 |
| 2 h Hydrolyse | 17,4 | 0,3 | 1,4 | 80,9 | 97,9 |
| 24 h Hydrolyse | 17,5 | 0,3 | 1,3 | 80,9 | 98,1 |

| Ansatz 2 | | | | | |
|---|---|---|---|---|---|
| 1 h Hydrolyse | 17,3 | 0,3 | 1,8 | 80,6 | 97,5 |
| 2 h Hydrolyse | 17,4 | 0,4 | 1,7 | 80,5 | 97,4 |
| 24 h Hydrolyse | 18,0 | 0,3 | 1,1 | 80,6 | 98,3 |

### Ergebnis:

Sowohl mit Novozym 525 wie auch mit Lipase G wird eine Erhöhung des Triglyceridgehaltes erreicht.

### Beispiel 5

### Synthese von Middle Chain Triglycerid (MCT) gekoppelt mit selektiver Hydrolyse der Partialglyceride und destillativer Aufreinigung des MCT

In einen Kolben werden 1050 g Caprylsäure, 200 g Glycerin, 50 g immobilisierte Candida antarctica B Lipase und 2 g Natriumcarbonat vorgelöst in 5 g Wasser gegeben. Bei einer Temperatur von 60 °C und einem Vakuum von 5 mbar wird der Ansatz unter Rühren und Stickstoffbegasung inkubiert. Nach 24 h wird das Vakuum abgestellt und eine Probe wird zur gaschromatographischen Analyse entnommen. Das immobilisierte Enzym wird abfiltriert. Zum Ansatz werden 200 g Wasser und 7 ml Candida antartica B Lipase in flüssiger Formulierung gegeben und der Ansatz wird für 2 h unter Rühren bei Raumtemperatur inkubiert. Nach 2 h wird der pH-Wert mit 0,1 M HCl auf 3,0 eingestellt und die Wasserphase wird über Zentrifugation separiert. Die Ölphase wird mit 200 g Wasser gewaschen. und die wässrige Phase wird erneut über Zentrifugation getrennt. Dann werden Caprylsäure und Monoglyceride destillativ entfernt. Dazu wird bei einer Temperatur von 190 °C bei einem Vakuum von 1 mbar über einen Dünnschichtverdampfer destilliert. Das Sumpfprodukt wird filtriert und einer Analyse unterzogen. Das Sumpfprodukt wird anschliessend einer Bleichung mit Bleicherde und Aktivkohle sowie einer zeitgleichen Raffination mit konzentrierter Natronlauge unterzogen.

**Tabelle 5: Synthese von Middle Chain Triglycerid (MCT) gekoppelt mit selektiver Hydrolyse der Partialglyceride und destillativer Aufreinigung des MCT**

| **Probe** | **Fettsäure [%]** | **Mono- glycerid [%]** | **Diglycerid [%]** | **Triglycerid [%]** | **% TG / Gesamtglyc.** |
|---|---|---|---|---|---|
| | | | | | |
| Ausgangsgemisch | 100 | 0 | 0 | 0 | 0 |
| 6 h Synthese | 32,3 | 1,4 | 34,0 | 32,1 | 47,6 |
| 24 h Synthese | 14,3 | 0 | 3,3 | 82,3 | 96,1 |
| 2 h Rückhydrolyse | 14,8 | 0,2 | 0,9 | 84,2 | 98,7 |
| Destillation Sumpf | 0,4 | 0 | 0,9 | 98,6 | 99,1 |
| Muster nach Raffination | 0 | 0 | 1,2 | 98,8 | 98,8 |

| | **Sâurezahl** | **OH-zahl** | **Farbe nach yellow** | **Lovibond 1 red** | |
|---|---|---|---|---|---|
| Destillation Sumpf | 0,8 | | 0,7 | 0,4 | |
| Muster nach Raffination | 0,2 | 1,3 | 0,2 | 0,1 | |

### Ergebnis:

Über enzymatische Rückhydrolyse gekoppelt mit Destillation und Raffination ist ein sehr hoher Triglyceridgehalt erreichbar, so dass auch die hohen OH-Zahl Anforderungen kosmetischer MCT Produkte erreicht werden.

## Patentansprüche

1. Verfahren zur enzymkatalysierten Synthese von Estern von Fettsäuren mit hohem Triglyceridanteil bei dem man
(a) eine Synthese der Fettsäuren oder ihrer Alkylester mit Glycerin in ihre Triglyceride in Gegenwart eines Enzyms unter Vakuum und wasserarmen Bedingungen durchführt
(b) im Anschluss die im Schritt a) ebenfalls entstandenen Partialglyceride in Gegenwart eines Enzyms nach Zugabe von Wasser unter Normaldruck rückhydrolysiert und
(c) Fettsäuren bzw. Alkylester und Glycerin destillativ oder über Raffination vom Triglycerid abtrennt,
wobei man kurzkettige Fettsäuren mit 6 bis 12 Kohlenstoffatomen und/oder mehrfach ungesättigte Fettsäuren auswählt aus der Gruppe, die gebildet wird von Docosahexaensäure, Eicosapentaensäure, Linolsäure und konjugierter Linolsäure in Form der freien Säure oder in Form der Methyl- oder Ethylester einsetzt, und wobei man immobilisierte Candida antarctica B Lipase sowohl für Synthese als auch Rückhydrolyse einsetzt.

2. Verfahren nach Anspruch 1, **dadurch gekennzeichnet, dass** man die Synthese (Schritt a) und die selektive Rückhydrolyse (Schritt b) im selben Reaktor (Eintopfverfahren) durchführt.

3. Verfahren nach mindestens einem der Ansprüche 1 bis 2, **dadurch gekennzeichnet, dass** man die Lipase in immobilisierter und / oder chemisch modifizierter Form einsetzt.

4. Verfahren nach mindestens einem der Ansprüche 1 bis 3, **dadurch gekennzeichnet, dass** man die Synthese (Schritt a) bei einem Vakuum von mindestens 200 mbar und die selektive Rückhydrolyse (Schritt b) bei Normaldruck durchführt.

5. Verfahren nach mindestens einem der Ansprüche 1 bis 4, **dadurch gekennzeichnet, dass** man die selektive Rückhydrolyse (Schritt b) bei mehr als 1 % Wasser und maximal 50 % Wasser im Reaktionsansatz bezogen auf den Gesamtansatz durchführt.

## Claims

1. A process for the enzyme-catalyzed synthesis of esters of fatty acids with a high triglyceride content, in which
(a) a synthesis of the fatty acids or alkyl esters thereof with glycerol to form their triglycerides is carried out in vacuo in the presence of an enzyme under low-water conditions,
(b) the partial glycerides also formed in step (a) are then back-hydrolyzed under normal pressure in the presence of an enzyme after the addition of water and
(c) fatty acids or alkyl esters and glycerol are separated from the triglyceride by distillation or refining,
wherein short-chain fatty acids with 6 to 12 carbon atoms and/or polyunsaturated fatty acids selected from the group consisting of docosahexaenoic acid, eicosapentaenoic acid, linoleic acid and conjugated linoleic acid in the form of the free acid or in the form of the methyl or ethyl esters, are used, and wherein immobilized Candida antarctica B lipase is used both for the synthesis and the back-hydrolysis steps.

2. A process as claimed in claim 1, **characterized in that** the synthesis (step (a)) and the selective back-hydrolysis (step (b)) are carried out in the same reactor (one-pot process).

3. A process as claimed in at least one of claims 1 and 2, **characterized in that** the lipase is used in immobilized and/or chemically modified form.

4. A process as claimed in at least one of claims 1 to 3, **characterized in that** the synthesis (step (a)) is carried out under a vacuum of at least 200 mbar and the selective back-hydrolysis (step (b)) is carried out under normal pressure.

5. A process as claimed in at least one of claims 1 to 4, **characterized in that** the selective back-hydrolysis (step (b)) is carried out with more than 1% water and at most 50% water in the reaction mixture, based on the mixture as a whole.

## Revendications

1. Procédé pour la synthèse d'esters, catalysée par une enzyme, d'acides gras à forte teneur en triglycérides, dans lequel
(a) on effectue une synthèse des acides gras ou de leurs esters alkyliques avec le glycérol en leurs triglycérides, en présence d'une enzyme, sous vide et dans des conditions pauvres en eau
(b) ensuite on rétro-hydrolyse les glycérides partiels formés également dans l'étape a), en présence d'une enzyme, après addition d'eau, sous la pression normale et
(c) on sépare les acides gras ou les esters alkyliques et le glycérol du triglycéride par distillation ou par raffinage,
dans lequel on utilise des acides gras à courte chaîne ayant de 6 à 12 atomes de carbone et/ou des acides gras plusieurs fois insaturés, choisis dans le groupe qui est constitué par l'acide docosahexaénoïque, l'acide eicosapentaénoïque, l'acide linoléique et l'acide linoléique conjugué, sous forme de l'acide libre ou sous forme de l'ester méthylique ou éthylique, et dans lequel on utilise aussi bien pour la synthèse que pour la rétro-hydrolyse la lipase B de *Candida antartica* immobilisée.

2. Procédé selon la revendication 1, **caractérisé en ce qu'**on effectue la synthèse (étape a) et la rétro-hydrolyse sélective (étape b) dans le même réacteur (procédé en un seul récipient).

3. Procédé selon au moins l'une des revendications 1 et 2, **caractérisé en ce qu'**on utilise la lipase sous forme immobilisée et/ou modifiée chimiquement.

4. Procédé selon au moins l'une des revendications 1 à 3, **caractérisé en ce qu'**on effectue la synthèse (étape a) sous un vide d'au moins 200 mbars et la rétro-hydrolyse sélective (étape b) sous la pression normale.

5. Procédé selon au moins l'une des revendications 1 à 4, **caractérisé en ce qu'**on effectue la rétro-hydrolyse sélective (étape b) avec plus de 1 % d'eau et au maximum 50 % d'eau dans le mélange réactionnel, par rapport au mélange total de départ.
